(19) Europäisches Patentamt
European Patent Office
Office européen des brevets

(11) **EP 3 730 058 A1**

(12) **EUROPEAN PATENT APPLICATION**

(43) Date of publication:
**28.10.2020 Bulletin 2020/44**

(51) Int Cl.:
*A61B 8/02* (2006.01)        *A61B 8/08* (2006.01)

(21) Application number: **19170857.7**

(22) Date of filing: **24.04.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **WOHLSCHLAGER, Markus Silvester
5656 AE Eindhoven (NL)**
• **FRANCK, Christoph Florian
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **FETAL ULTRASOUND PROCESSING UNIT FOR SEPARATING HEART RATE SIGNALS**

(57)  A processing unit and method are provided for processing fetal Doppler ultrasound data to extract a set of signals representative of different distinct heart rate signal sources, i.e. maternal heart rate and fetal heart rate. Doppler data is received corresponding to a plurality of different depth zones (16a, 16b, 16c) in the fetal region and, these processed into a set of input channels, each corresponding to a different depth. These are then processed successively by a PCA algorithm followed by an ICA algorithm, which work to unmix the multiple heart rate sources present in each of the input channels, and derive a set of output signals from the ICA which can be taken as representative of separate heart rate sources.

FIG. 2

EP 3 730 058 A1

**Description**

FIELD OF THE INVENTION

The invention provides an ultrasound processing unit for use in distinguishing different heart rate signals within ultrasound Doppler data.

BACKGROUND OF THE INVENTION

Electronic fetal monitoring (EFM) typically uses Doppler ultrasound to acquire a pulse (heart rate) signal from a fetus in utero during pregnancy and labor. The fetal heart rate (FHR) is calculated using the acquired pulse signal.

The Ultrasound (US) Doppler transducer used for this purpose typically utilizes an unfocused, approximately cylindrical ultrasound beam field. An extent of the beam volume is defined by a characteristic reception time window. During this window the US transducer is set to acquire reflected signals from any moving anatomical structures.

Depending on the orientation and the size of the beam field, it is possible that more than one pulse signal source may be present: one corresponding to the maternal pulse rate, and one (or more, in the case of multiple pregnancies) fetal pulse rate sources.

In order to focus on one signal source alone, conventionally the receive window is actively adjusted to alter the particular depth and volume from which US signals are obtained, and thus which is observed.

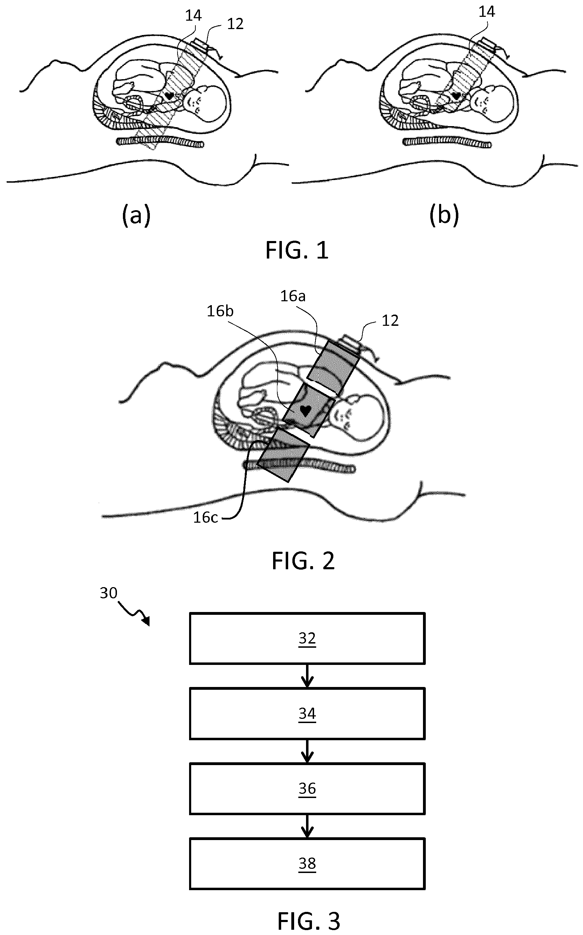

This is illustrated in Fig. 1 which schematically depicts US observation of the fetal area at different depths. Fig. 1(a) schematically depicts a deep observation area, and Fig. 1(b) depicts a shallower observation area. For Fig. 1(a), the timing and duration of the receive window of the ultrasound transducer 12 is adjusted, relative to the timing and duration of the US transmission, so that US reflections are detected from a greater depth. As a result a deeper observation volume 14 is obtained. Conversely, in Fig. 1(b), the receive window is adjusted so that US reflections from a shallower depth are detected, resulting in a shallower depth volume 14.

State of the art EFM units may use an iterative approach to determine an optimum depth range to use. In particular, the iterative approach may seek the depth range that contains the largest amount of signal strength, while excluding areas that contribute noise or weaker signal components. In Fig. 1 for example, the shallower depth range of Fig. 1(b) provides the stronger signal since it encompasses the fetal heart, but excludes any noise which might be present at depths below the fetal heart.

The iterative approach may typically use two separate receive windows with independently adjustable start and stop depths. One window is used for the actual signal acquisition. The other window is used to probe whether signal intensity may be increased by including a larger depth range, and/or, whether reducing the depth range will significantly reduce signal intensity. Often, a receive window range that is as small as possible is preferred, as smaller windows typically result in reduced noise.

This iterative approach however is not able to discriminate between different pulse signal sources, e.g. two fetal hearts; or maternal and fetal heart. Rather, it will typically encompass all detectable pulse rate sources within its calculation, and attempt to incorporate all of these within the selected depth window. As a consequence, the adjustment procedure may result in acquisition of a Doppler ultrasound signal which is a mixture of two or more pulse rate sources. As a result, the FHR (fetal heart rate) calculation performed on the basis of this signal may fail or lead to an erroneous measurement. Typical FHR calculation algorithms are based on autocorrelation. These are sporadically unable to generate an FHR that reliably corresponds to a single present heart rate source. Instead, the calculated FHR number is erroneous and not related to any single physiological signal.

There would be advantage therefore in providing an improved Doppler ultrasound processing approach, capable of distinguishing different heart rate sources within the input data.

SUMMARY OF THE INVENTION

The invention is defined by the claims.

According to examples in accordance with an aspect of the invention, there is provided an ultrasound processing unit, for use in fetal monitoring, for distinguishing between different heart rate sources within received Doppler ultrasound data, the unit configured to:

receive input Doppler ultrasound data, including data corresponding to a plurality of different depths within a uterus region of a subject, and extract from the data a set of input signal channels, each representative of a different tissue depths within the subject;
perform a principal component analysis, PCA, procedure, configured to identify one or more linear combinations of the input signal channels which are statistically uncorrelated with one another, the linear combinations defining, when composed, a set of first output signals, and
perform an independent component analysis, ICA, procedure, configured to identify one or more linear combinations of said first output signals which are statistically independent from one another, said linear combinations defining a set of second output signals. Due to the effect of the combined PCA and ICA procedures, the resulting second output signals can each reliably be taken as corresponding to a single heart rate signal source.

The invention utilizes a different approach to the standard iterative procedure, in which the depth is iteratively adjusted. Instead, a plurality of different depth signals are acquired together, and these are then mathematically processed to identify and extract the individual underlying heart rate signals present within them.

Doppler signals are obtained from a plurality of, preferably spatially adjacent, depth regions, for instance by gating the incoming reflection signal over series of temporally successive windows. The resulting input depth channels may each contain a mixture of the multiple heart rate sources.

The particular lengths and depths (i.e. the depth start and end points) of the different depth windows may be determined by the controller adaptively in some examples, for instance determined based on one or more parameters related to the subject. For example, the depth segments that are captured may be determined based on a BMI of the subject (thus, to account for different fat layer thicknesses of subjects). In other examples, the depth windows sampled may have fixed lengths and depth locations.

The PCA and ICA algorithms are mathematical techniques used in signal analysis for separating out mixtures of signal sources. By providing a plurality of input mixed signals (the input signal channels), the combination of these algorithms enables extraction of a set of second output signals, each of which corresponds to a single heart rate source.

The combined application of the PCA procedure and ICA procedure leads to more complete unmixing of the original heart rate sources present within the data than would be achievable with either of the two processes alone. As a result, the set of second output signals can be taken to be reliably representative of different heart rate sources

Each linear combination of input signals identified by the PCA procedure corresponds to a single first output signal. The PCA procedure may identify the linear coefficients (or weightings) which define each linear combination, and/or may generate each first output signal by combining the relevant signals which form each one, with the weightings identified.

The PCA procedure is configured to identify one or more linear combinations of the input signal channels which are statistically uncorrelated with one another.

Statistical correlation is a term of the art. In particular, statistical correlatedness is a well-defined term in the field of mathematical statistics. Two random variables X and Y are statistically uncorrelated if the expected value of their product is equal to the product of their expected values:

$$E\{X \cdot Y\} = E\{X\} \cdot E\{Y\}$$

In the context of the present invention, the variable X would be a first one of the identified linear combinations of input signal channels, and the variable Y would be a second one of the identified linear combinations of input signal channels.

This definition is readily expanded to vectors of random variables. In particular, the first term in the equation above becomes the expectation value of the outer, or dyadic, product of the random vectors x and y and is the cross-correlation matrix of the two vectors:

$$E(\mathbf{x} \cdot \mathbf{y}^{\mathrm{T}}) = E(\mathbf{x}) \cdot E(\mathbf{y}^{\mathrm{T}})$$

For the vector version, the expected value of the outer product of vectors x and y must be equal to the outer product of their individual expected values for the vectors to be uncorrelated. The outer product of two vectors is a matrix. The superscript $^{\mathrm{T}}$ denotes a transpose of the relevant matrix in the above expression.

In the context of the present invention, the PCA algorithm may generate as an output a single vector, z, whose elements are constituted by the set of first output signals (the set of linear combinations of input signal channels). The aim of the PCA algorithm, as discussed, is to make the individual components (channels) of such a (single) output vector uncorrelated.

To apply the above (general) uncorrelatedness condition for a single output vector to be generated by the PCA algorithm, the condition may be expressed in slightly different form. Uncorrelatedness of the individual elements of a single output vector, z, of output signal channels is achieved when all elements outside the diagonal of the covariance matrix

$$\mathbf{C}_{\mathbf{z}} = E((\mathbf{z} - \mathbf{m}_{\mathbf{z}}) \cdot (\mathbf{z} - \mathbf{m}_{\mathbf{z}})^{\mathrm{T}})$$

are zero (where $\mathbf{m}_{\mathbf{z}}$ is the vector of mean values of the elements of z). The diagonal elements are nonzero for any component that does not have a constant value, as the diagonal elements contain the variances of each channel.

The definition of statistical correlatedness may be found for example in the book: Appo Hyvärinen, Juha Karhunen, Erkki Oja, "Independent Component Analysis", John Wiley & Sons, Inc., 2001.

Statistical independence is also a well-defined term in mathematical statistics. Two variables, x, y, are statistically independent if their joint probability (the probability of observing certain combinations of values) factorizes into a product of individual probabilities:

$$p_{x,y}(x, y) = p_x(x) \cdot p_y(y)$$

This effectively means that knowledge of the value of one of the variables x, y, gives no information at all about the value of the other variable.

Statistically independent variables are always uncorrelated, but uncorrelated variables are not necessarily independent. By way of example, consider two random variables where one variable is always zero if the other variable is different from zero. The two variables are uncorrelated, as their product is always equal to zero, but not independent, as knowing that one variable is nonzero allows the deduction that the other variable is zero.

The PCA procedure may be configured to identify the linear combinations of said input signals that result in first output signals having a combined signal strength exceeding a defined threshold while being statistically uncorrelated with one another.

The threshold may be defined relative to an average or a maximum signal strength among the input signals, or among possible combined signals. For example, the threshold might be a signal strength 50% or 75% of the maximum signal strength among the possible combined signals.

The PCA algorithm thus selects the linear combinations which result in a largest combined signal strength, while remaining statistically uncorrelated.

The processing unit may be further configured to generate the set of second output signals in accordance with the identified linear combinations. This comprises combining the input signals together with the particular sets of weightings defined for each linear combination (each second output signal).

Signal combination is a routine process and the skilled person will be aware of means for achieving this.

The processing unit may be adapted to process the second output signals to derive from each a heart rate signal or heart rate measurement.

The processing unit may be further adapted to attribute to each of the second output signals a physiological source.

The processing unit here derives a physiological attribution for each of the second output signals by determining a physiological source of each signal.

The processing unit may determine for instance which of the output signals corresponds to the maternal heart rate and which to the fetal heart rate(s).

The attribution may be based on comparison of one or more signal characteristics of the second output signals.

The attribution may be performed based on one or more of: a weighted average of the depths within the subject to which the second output signals correspond, a pulse rate associated with the second output signal, and a spectral content of the signal.

The weighted average depth may be determined as a weighted average of the depths of the input signals which are included in the second output signal. This may be determined based on analysis of the input signals included in the first output signals of which the second output signal is composed. The average is preferably weighted according to the contribution of each input signal to the output signal, for example, if an output signal is composed to 75% of an input signal corresponding to 'depth range 20 cm' and 25% of an signal corresponding to 'depth range 15 cm', the weighted average would be given by (0.75 x 20 cm) + (0.25 x 15 cm) = 18.75 cm.

The PCA procedure is configured in general to provide as an input to the ICA procedure an indication of the identified linear combinations of the input signals.

In some examples, the PCA procedure may provide as an input to the ICA procedure a plurality of sets of linear coefficients which define said identified linear combinations of input signals. The PCA procedure may output a matrix, the elements of which are populated by the linear coefficients.

Additionally or alternatively, the PCA procedure may be configured to generate the set of first output signals in accordance with the identified linear combinations, and provide the signals as an input to the ICA procedure.

As noted above, according to one or more embodiments, extracting the set of input signal channels may comprise gating the input Doppler ultrasound data over a plurality of different temporal windows, for example over a series of temporally successive windows. The input signal channels may hence each correspond to a different captured ultrasound window. These may be termed 'depth windows' within this disclosure.

Examples in accordance with a further aspect of the invention provide an ultrasound apparatus comprising:

an ultrasound processing unit in accordance with any example or embodiment outlined above or described below, or in accordance with any claim of this application; and
one or more ultrasound transducers, operatively coupled to the ultrasound processing unit, for providing the input Doppler ultrasound data to the ultrasound processing unit.

The apparatus may comprise an ultrasound probe unit, the probe unit incorporating the ultrasound processing unit and the one or more ultrasound transducers.

The probe unit may for example have a housing, the ultrasound processing unit and the one or more ultrasound transducers being included within the housing.

Examples in accordance with a further aspect of the invention provide patient monitoring system comprising: an ultrasound processing unit in accordance with any example or embodiment outlined above or described below, or in accordance with any claim of this application; and a connection interface for connecting in use to an ultrasound transducer unit for receiving the input Doppler ultrasound data, or data based thereon.

The connection interface may be further coupled to the ultrasound processing unit to transfer the received ultrasound data.

The connection interface may be a wired connector, or may be a wireless connection interface for connecting to a wireless ultrasound probe.

The patient monitoring system may further comprise an ultrasound transducer unit operatively coupled to the ultrasound processing unit via said connection interface.

The transducer unit may be an ultrasound probe for example.

The ultrasound transducer unit may be only a transmit/receive unit, i.e. comprising one or more ultrasound transducers for transmitting and sensing ultrasound signals. Here, the ultrasound processing unit comprises all signal processing components, including components for digitization and demodulation of the analogue signals output by the transducer unit, to separate the different depth channels. In this case, analogue signals are communicated from the transducer unit to the ultrasound processing unit via the communication interface.

In other examples, the ultrasound transducer unit may additionally comprise local, or on-site, signal processing components for performing digitization and demodulation of the signals and to achieve separation of the depth channels. In this case, the resulting digital data representative of the separated signal channels is communicated from the ultrasound transducer unit to the ultrasound processing unit.

The patient monitoring system may include a base station or base unit, to which at least an ultrasound transducer unit, e.g. an ultrasound probe, can be connected. The base station may include a display for displaying results of the processing performed by the ultrasound processing unit.

The ultrasound processing unit may be comprised by the base station. Alternatively, the ultrasound processing unit may in some examples be comprised by the ultrasound transducer unit.

The patient monitoring system may include a base station connected or connectable with an ultrasound apparatus as described above, comprising an ultrasound probe integrating ultrasound transducers and the ultrasound processing unit.

The patient monitoring system may further include a controller adapted to control acquisition of ultrasound data by a connected transducer unit in use.

The controller may control transmit and receive circuits of the ultrasound transducer unit to acquire the ultrasound signals representative of different depths. The controller may control durations of, and timings between, transmit pulses and receive windows. The controller may control gating of the input Doppler signal data over defined time windows to thereby separate different input signal channels corresponding to different depths within the subject's tissue.

In certain examples, the different depths may correspond to different depth ranges within a single generated cylindrical ultrasound beam field.

In certain examples, the ultrasound transducer unit may comprise an array of individual ultrasound transmitters, and wherein a control means is configured to apply beamforming using the array, to control a directionality of a generated ultrasound beam. The beamforming may be controlled so as to acquire ultrasound signals from multiple different beam directions. Signals from multiple different depths within each beam may in this case be acquired. The ultrasound processing unit is configured in this case to extract a plurality of different depth channels from each beam. This approach allows a larger volume of tissue to be scanned with a single transducer unit, and at the same time generate a larger number of ultrasound channels for the PCA algorithm to process.

Examples in accordance with a further aspect of the invention provide an ultrasound processing method for use in distinguishing heart rate sources within received Doppler ultrasound data, the method comprising:

receiving input Doppler ultrasound data, including data corresponding to a plurality of different depths within a uterus region of a subject, and extracting from the data a set of input signal channels, each representative of a different tissue depth within the subject;

performing a principal component analysis, PCA, procedure, to identify one or more linear combinations of the input signal channels which are statistically uncorrelated with one another, the linear combinations defining, when composed, a set of first output signals, and

performing an independent component analysis, ICA, procedure, configured to identify one or more linear combinations of said first output signals which are statistically independent from one another, said linear combinations defining a set of second output signals.

The method may further comprise generating the set of second output signals in accordance with the identified linear combinations.

The method may further comprise processing the second output signals to derive from each a heart rate signal or heart rate measurement.

The method may further comprise attributing to each of the second output signals a physiological source.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a known approach for acquiring fetal heart rate data based on iteratively adjusting a depth of acquired signals;

Fig. 2 illustrates an approach according to embodiments of the present invention, based on acquiring signals for multiple adjacent depth zones;

Fig. 3 is a block diagram of steps performed by an example processing unit according to one or more embodiments;

Fig. 4 shows a workflow of an example processing unit according to one or more embodiments;

Fig. 5 illustrates the result of application of embodiments of the present invention to a set of mixed input signals; and

Fig. 6 shows an example ultrasound system according to one or more embodiments,

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a processing unit and method for processing fetal Doppler ultrasound data to extract a set of signals representative of different distinct heart rate signal sources, i.e. maternal heart rate and fetal heart rate. Doppler data is received corresponding to a plurality of different depths in the fetal region and, these processed into a set of input channels, each corresponding to a different depth. These are then processed successively by a PCA algorithm followed by an ICA algorithm, which work to unmix the multiple heart rate sources present in each of the input channels, and derive a set of output signals from the ICA which can be taken as representative of separate heart rate sources.

State of the art electrical fetal monitoring (EFM) approaches are often unsatisfactory for cases in which several pulse signals are present within the ultrasound pickup range (the volume of observation) of the ultrasound transducer. Erroneous fetal heart rate (FHR) readings are generated because known devices are not able to distinguish between the different heart rate sources, leading to mixing of the sources when taking the measurements. This can lead to unnecessary surgical interventions or adverse delivery outcomes.

Embodiments of the present invention provide an improved approach to fetal heart rate monitoring based on use of two key mathematical techniques: principal component analysis (PCA) and independent component analysis (ICA).

As noted above, known EFM ultrasound systems, use an iterative approach, which iteratively trials signal acquisitions from different depth regions, seeking a receive window with optimum signal strength.

The present invention applies a different approach in which, instead of using receive windows with adjustable depth parameters, the transducer acquires ultrasound data across a plurality of adjacent depth windows with fixed lengths. This may involve gating the incoming Doppler ultrasound signal over successive time windows (receive windows), thereby deriving a plurality of signals corresponding to successive, and adjacent, depth regions within the subject

Fig. 2 schematically illustrates acquiring ultrasound signals from an example set of three depth zones 16a, 16b, 16c. The signals from the different zones provide a plurality of depth channels which are together provided as a set of signal inputs to the PCA algorithm, followed by the ICA algorithm as will be explained below.

Examples in accordance with a first aspect of the invention provide an ultrasound processing unit for use in fetal monitoring for distinguishing different heart rate sources within received Doppler ultrasound data.

Fig. 3 shows a flow diagram illustrating in block diagram form the basic steps performed by the processing unit.

The processing unit is adapted to receive 32 input Doppler ultrasound data, including data corresponding to a plurality of different depths within a uterus region of a subject.

The processing unit then extracts 34 from the input data a set of input signal channels, each representative of a different tissue depth within the subject.

The unit then performs a principal component analysis, PCA, procedure 36, configured to identify one or more linear combinations of the input signal channels which are statistically uncorrelated. The linear combinations define a set of first output signals. The PCA procedure provides an indication of the identified linear combinations of input signals as an input to the ICA. This may be in the form of a set of linear coefficients, or weightings, of the input signals in some examples. The PCA procedure may comprise generating the first output signals based on the derived linear combinations. These may be provided as an input the ICA procedure in some examples, either instead of, or in addition to, the linear coefficients (weightings).

The unit then performs an independent component analysis, ICA, procedure 38, configured to identify one or more linear combinations of said first output signals which are statistically independent from one another, said linear combinations defining a set of second output signals. The set of second output signals are taken as representative of separate distinct pulse rate sources within the probed region. The ICA procedure may generate the set of second output signals in accordance with the identified linear combinations of the first output signals. These may be provided as an output. These may be displayed on an associated display device for example, for observation by a user, e.g. a clinician.

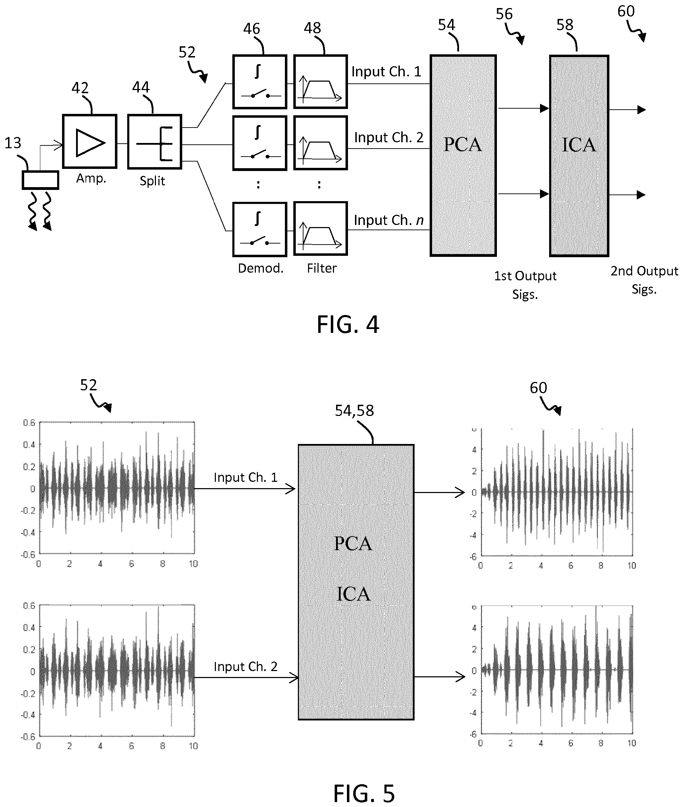

Fig. 4 schematically depicts in more detail an example workflow for a processing unit according to one or more embodiments. Although certain functions are shown as performed by separate components, this is for illustration only in this figure. It is to be understood that in general the functionality performed by these components may be performed by a different combination of one or more components, for instance a single unitary processor in some examples.

Doppler ultrasound data is first obtained using an ultrasound transducer unit. The transducer unit may comprise a plurality of ultrasound transducers or a single transducer. The received ultrasound data is representative of a plurality of different depths within the targeted body. From this data, there may be extracted a plurality of input channels corresponding to signals for different channels.

In more detail, in operation, ultrasound pulses may transmitted by an ultrasound receive/transmit unit 13 into the body being probed, i.e. the uterus region of the subject. The pulses are transmitted at a defined frequency over a defined transmit window, or recurrent set of transmit windows. The receive/transmit unit comprises one or more ultrasound transducers for generating and sensing ultrasound signals. It is a form of ultrasound transducer unit, but without comprising signal processing components (which are comprised externally to the unit in this example).

Reflected ultrasound signals are then received back at the ultrasound receive-transmit unit 13. Reflections will be received at the receive-transmit unit at different time points depending upon the depth from which the signal is reflected. As the propagation speed of ultrasound in tissue is known (approximately 1000 meters/second), the time delay between transmission and reception may be mapped to the distance the ultrasound pulse has travelled. This distance is then proportional to the depth.

In some examples, signals are transmitted in a single direction, and ultrasound signals received and gated corresponding to different depths within said single cylindrical beam field.

In further examples, the ultrasound transducer unit may comprise an array of individual ultrasound transmitters, and wherein a control means is configured to apply beamforming using the array, to control a directionality of a generated ultrasound beam. The beamforming may be controlled so as to acquire ultrasound data from multiple different beam directions. Signals from a plurality of different depths within each directional beam may in this case be acquired. The ultrasound processing unit may be configured in this case to extract a plurality of different depth channels (according to the procedure described below) from each beam. This approach allows a larger volume of tissue to be scanned with a single transducer unit, and at the same time generate a larger number of ultrasound channels for the PCA algorithm to process.

In either case, the input data may be amplified by an amplifier 42, and then split 44 into a plurality of separate input channels 52, corresponding to the different depth regions within the subject.

Signals corresponding to different depths may be separated 44 for example by gating the incoming signal over different temporal receive windows, each gated signal then providing a different input signal channel 52 corresponding to a different depth.

In some examples, the duration of, and timing between, transit pulses and receive windows can be adjusted so that receive signals from specific desired depths can be obtained, these then being gated over the appropriate time windows to provide different depth signals on each depth channel 52.

Optionally, the gating pulses for the channels may be produced by digital logic (e.g. a microcontroller, FPGA or similar) included the receive-transmit (or ultrasound transducer) unit 13. The gated signal may subsequently be sampled with an analog-to-digital converter. A relatively low sampling rate may be used (for example several hundred to several thousand Hz).

The skilled person in this field will be aware of numerous approaches to gating signals to extract channels corresponding to different depths within the probed body.

The different gated depth signals each provide a different input signal channel 52, as shown in Fig. 4.

Pre-processing steps 46, 48 are applied to each input signal channel 52. These may be applied after separating the different input signal channels 52 (as in the illustrated example of Fig. 4), or before.

In particular, a demodulation and signal integration 46 may be applied to the input signals of each input signal channel 52. Demodulation generates a signal with a frequency equal to the Doppler (frequency) shift of the measured Doppler signal, compared to the original transmitted signal.

Bandpass filtering 48 may be applied to each input signal channel 52. The filtering is configured to select the frequency component of the incoming signal within the frequency range expected for the heartbeat measurement. This ensures only the relevant frequency component of the data is retained, reducing overall noise.

An envelope demodulator may additionally be applied in some examples (not shown). This extracts for each input signal channel 52 an envelope signal corresponding to the change in signal strength (e.g. intensity or variance), as a function of time, for the selected (filtered) frequency range.

In certain examples, the demodulation 46 function might be incorporated into a digital microprocessor comprised by the ultrasound transducer unit 13 In this case, the reflection of the transmitted pulse may be sampled with an analog-to-digital converter at a high sampling rate (several times the frequency of transmitted ultrasound pulse, e.g. several MHz), and digital logic or software in the transducer unit 13 may demodulate the received signal and calculate a signal value for each depth channel. This may remove the need for gating pulses and analog demodulation circuitry.

Providing a digital microprocessor capable of analogue to digital conversion at the required resolution and speed to provide processing synchronously with signal acquisition may be challenging with current microprocessor technology. In alternative examples, splitting of the input signal channels may be performed instead using a dedicated analogue to digital (A/D) converter included in the ultrasound transducer unit 13. The A/D converter should in this case operate synchronously, meaning the ultrasound frequency matches (is preferably identical to) the A/D conversion frequency.

Following pre-processing, each input signal channel (depth channel) is provided as an input to a principal component analysis (PCA) algorithm 54.

In summary, this algorithm determines linear combinations (weighted sums) of the input signal (depth) channels that capture the largest strength (variance) of the signal, while being statistically uncorrelated. These linear combinations correspond, when composed, to a set of first output signals 56.

The output of the PCA algorithm 54 provides an indication of the total number of heartbeat signal sources present in the collection of depth signals. However, the first output signals 56 of the PCA may still contain mixtures of the original heartbeat signal sources. The PCA alone therefore may not be sufficient to fully separate the different heartbeat sources.

PCA can be used to reduce the initial number of input channels 52 (which may be very large if the ultrasound transducer unit uses many depth channels) to the number of uncorrelated, strong pulse rate signals (i.e. the first output signals 56). The set of first output signals 56 typically numbers fewer than the total number of input signal channels 52.

Reducing the number of channels simplifies subsequent processing steps and excludes mathematical subspaces of the depth channel vector that contain only noise. The PCA algorithm effectively performs a first stage of unmixing of the different pulse signal sources.

PCA can be performed with a number of algorithms, including, by way of example, online neural network algorithms or other machine learning algorithms. PCA in general may be considered a subset (or a specific technique) of machine learning.

By way of example, chapter 6 ("Principal Component Analysis and Whitening"), in the book "Independent Component Analysis" by Hyvärinen, Karhunen and Oja describes in detail procedures for implementing suitable principal component analysis procedures. The chapter in particular describes several elements of PCA, including one-by-one extraction of principal components and parallel extraction of multiple principal components; sample-by-sample and batch mode algorithms; and methods for determining the number of components that should be extracted.

The first output signals 56 are then provided as an input to a more complex independent component analysis (ICA) procedure 58. This technique determines linear combinations of the first output channels 56 that are statistically independent. This results, in the ideal case, in a number of second output signal channels 60 where each second output signal mostly contains only the Doppler ultrasound signal of one pulse signal source in the abdomen. The ICA algorithm completes the unmixing of the original pulse signal sources.

The PCA algorithm and ICA algorithm will now be explained in more detail.

The PCA algorithm 54 processes the input signal channels 52 and may provide as an output a set of weight vectors (or linear coefficients) that describe how to (linearly) combine the input channels 52 to form the first output channels 56. Preferably, the PCA also outputs the first output channels 56 themselves, which may or may not consist of a smaller number of channels than the set of input channels 52. The PCA may output a vector of the output channels.

In vector/matrix form, PCA computes

$$z(t) = V * x(t),$$

with x being a column vector of n elements corresponding to the input signal channels 52, z being a column vector of m elements corresponding to the output channels, and V being a matrix with m rows and n columns which makes the elements of z statistically uncorrelated and usually also normalizes their statistical variance to 1.

If the output signals are both made uncorrelated and their variance is normalized, the process is otherwise known as "whitening".

PCA algorithms determine a value of V and z, given x as input. There is usually no single unique solution for V and z; a PCA algorithm finds one of infinitely many possible solutions that make the elements of z uncorrelated. PCA works by considering the variances and the cross-correlations (the so-called second order statistics) of the input signals 52 only, so it can achieve uncorrelatedness, but not complete statistical independence of the elements of z.

As a simple example, there may be provided three input channels in x, and prior knowledge that there exist a total of two heart rate signal sources present in the channels of x at different intensities. The problem is to seek two output channels 56, and V in the form of a 2-by-3 matrix.

If the first heart rate signal source is present in the first and second input channel 52 of x at equal intensity, and the second heart rate signal source is present only in the third input channel 52 of x, a PCA algorithm should result in a V with the form:

$$V = \begin{bmatrix} 1 & 1 & 0 \\ 0 & 0 & 1 \end{bmatrix}$$

In this particular example, the PCA algorithm would in fact also achieve complete separation of the two sources (since they were not really mixed in the first place).

It is noted that as the maximum number of independent signal sources is usually known in fetal monitoring applications, advantageously, this information may be used to reduce the complexity of the PCA procedure, reducing the number of dimensions which the PCA algorithm is required to consider in the processing. The algorithm itself is not required to determine in this case how many output channels it should produce. This may increase the speed of the algorithm.

In particular, due to the matrix operations involved in the algorithm, the computational complexity increases with the third power of the number of channels. Hence, reducing the problem from four (or more) channels to two or three (in case of a twin pregnancy) significantly reduces the computational requirements of the algorithm.

This simplification may be pre-programmed in the algorithm, or it may be provided as an adjustable setting of the algorithm. For instance the processing unit may be configured to receive a user input representative of a total number of heart rate source signals, this being determined for instance based on whether there is a single or double (or more) pregnancy.

PCA is a well-known procedure within the field of signal analysis, and the skilled person will be aware of the principles behind it, and of detailed means for implementing the procedure. By way of example the book, "Handbook of Blind Source Separation" by Comon and Jutten, provides more information on PCA algorithms which may be applied in accordance with embodiments of the present invention.

The Independent Component Analysis (ICA) algorithm may also output a set of weight vectors (for example represented in matrix form) defining the derived set of linear combinations of the first output signals. It may also output a vector of the second output signals.

In general, an ICA algorithm finds a matrix W so that the elements of y(t)

$$y(t) = W * z(t)$$

are statistically independent. z(t) is the output of the PCA/whitening algorithm.

The ICA algorithm looks beyond the second-order statistics considered by the PCA algorithm, and considers further statistical properties such as kurtosis, signal entropy, or mutual information of the channels of y, as these properties quantify statistical dependence/independence.

In general, ICA algorithms may be constructed by choosing a cost function (e.g. kurtosis, signal entropy, or mutual information) that is to be minimized or maximized by finding a suitable W, and choosing an optimization algorithm for the minimization/maximization (e.g. gradient descent, stochastic gradient descent, Newton's method).

As the cost function is nonlinear, the algorithm is required to iterate over several approximate solutions in order to find the W that minimizes/maximizes the cost function, taking applicable constraints into account (such constraints can be used to prevent the optimization algorithm from simply setting W to zero to minimize the cost function for example, or by letting the values of W increase without bounds to maximize the cost function).

After application of the PCA and ICA procedures, the matrices W and V may optionally also be combined into an "unmixing matrix" B, where

$$y(t) = W * z(t) = W * V * x(t) = B * x(t)$$

Here, B directly describes the linear combinations of x that form the output channels of y. B provides information indicative of whether and how strongly the channels of x appear as components of each output channel in y.

ICA is a well-known procedure within the field of signal analysis, and the skilled person will be aware of the principles behind it, and of detailed means for implementing the procedure. Further details on example ICA algorithms may be found for example in the book: Appo Hyvärinen, Juha Karhunen, Erkki Oja, "Independent Component Analysis", John Wiley & Sons, Inc., 2001.

The second output signal channels 60 of the ICA may be taken as representative of individual heart rate signal sources.

The second output signals 60 of the ICA may subsequently be provided as an input to a heart rate calculation algorithm. Algorithms for deriving a heart rate measurement or signal based on a Doppler ultrasound signal are known in the art. Some for example are based on autocorrelation.

By way of example, one suitable example heart rate calculation algorithm is outlined in the document US 4,403,184. This example is based on autocorrelation. Using autocorrelation to determine the frequency of a repeating signal is an established technique in the field.

Since each second output signal channel is predominantly composed of only one heart rate signal source, occurrence of erroneous FHR readings due to mixed signals may be very significantly reduced compared to existing solutions.

According to one or more advantageous embodiments, the processing unit may be further configured to derive a physiological source attribution for each of the second output signals 60, i.e. to determine whether each signal corresponds to a material heart rate or a fetal heart rate.

This attribution process may be based on a comparative approach comprising comparing one or more properties of the second output signals.

For example, properties of the different second output signals 60 such as the average depth of an identified signal source, the pulse rate, the spectral content of the signal, may be compared, and the results used to inform an attribution. There may be stored known average or typical values of one or more of these properties for maternal and fetal heart rate signals respectively, and these used as references to determine an attribution for each of the second output signals 60. Other properties such as maternal ECG or SpO2 pulse rates may additionally be used to inform the attribution process in some examples.

A comparative approach of classifying the signal sources (comparing signal properties of the various output signals 60) is a simpler approach than considering each source in turn and analyzing it to determine an attribution.

The effectiveness of the approach of the present invention is illustrated by Fig. 5. The two input signals channels 52 are illustrated, representative of different depths within the probed subject: input signal channel 1 ("input Ch. 1") and input signal channel 2 ("input Ch. 2"). Each represents a mixture of the underlying heart rate sources.

Processing each of the two signals with a conventional autocorrelation method would be unlikely to yield an accurate heart rate reading.

Treating both input signals 52 instead with the PCA ICA method, 54, 58 significantly improves the output signal quality and eliminates any interference between the heart rate signal sources.

The combination 54, 58 of the PCA and ICA algorithms generates two output signals 60, representative of the two underlying heart rate signal sources, HR1 and HR2. The elimination within each of interfering components of the other channel avoids falsification and miscalculation when subsequently calculating the beat to beat value of the heart rate.

As discussed above, the approach of the present invention provides a significant improvement over existing approaches to detecting fetal heart rates.

Distinguishing between maternal and fetal heart rates is a difficult problem with conventional processing approaches. Often the signals can vary in amplitude and strength at different times, leading to confusion in algorithms designed for separating the signals.

Situations with amplitude equality between the fetal heart rate signal and the interfering maternal heart rate signal can often occur with current EFM technology and known depth selection algorithms.

Due to signal superposition the true peak position of the fetal heart beat may be effectively blurred, leading to an inaccurate heat rate calculation. Episodes with strong fetal and maternal signal mixtures often show a reduction or an increase of the calculated heart rate, which can be incorrectly interpreted as a deceleration or respectively an acceleration of heart rate. This can lead to inappropriate medical interventions.

Current EFM technology also requires an operator to use care when placing EFM transducers on the maternal abdomen in order to minimize the risk of acquiring a plurality of independent pulse signal sources (either maternal and fetal, or multiple fetal sources in the case of multiple pregnancies). This requires skilled training for the operator and also increases the time required for EFM procedures.

Embodiments of the present invention provide improvements in the EFM technology in a number of different ways. By separating the volume covered by the ultrasound beam into several depth slices (as described above) and using PCA and ICA to isolate independent pulse signals, the risk of calculating erroneous pulse rates due to mixed pulse signals is greatly reduced. This reduces the occurrence of unnecessary surgical interventions and adverse outcomes.

In addition, the increased robustness of the signal separation process of the present invention means that fewer constraints are required when positioning the ultrasound transducer on the maternal abdomen. This renders the EFM system easier and more convenient to use and also improves patient comfort.

Additionally, embodiments of the present invention are able to determine the total number of independent pulse signal sources within the ultrasound field of view, and analyze each of the source signals separately. This can be used to provide both a maternal pulse rate in addition to the fetal pulse rate, or to monitor multiple fetuses with a single transducer.

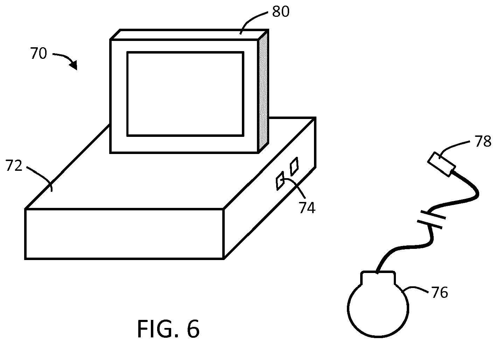

Examples in accordance with a further aspect of the invention provide a patient monitoring system. An example patient monitoring system 70 in accordance with one or more embodiments is shown in Fig. 6.

The patient monitoring system 70 comprises an ultrasound processing unit in accordance with any example or embodiment outlined above or described below, or in accordance with any claim of this application. In the example shown the ultrasound processing unit is incorporated internally within a base station unit 72. In other examples however, the ultrasound processing unit may be incorporated locally within an ultrasound transducer unit 76 with which the base station unit is connected or connectable.

The patient monitoring system 70 further comprises a connection interface in the form of an input connector port 74 for connecting in use to an ultrasound transducer unit 76 for receiving the input Doppler ultrasound data, or data derived therefrom. An example ultrasound transducer unit 76 for connecting in use to the base station is shown in Fig. 6. The transducer unit comprises an output connector 78 shaped to engage with the input connector 74 of the base station.

Where the ultrasound processing unit is included in the base station 72, the input connector 74 may be coupled to the ultrasound processing unit to transfer the received ultrasound data.

The connector 74 is shown as a wired connector port in Fig. 6. In other examples, the connector may comprise a wireless connection interface for connecting to a wireless ultrasound probe.

The patient monitoring system 70 may further comprise an ultrasound transducer unit 76 coupled to said input connector. The transducer unit may be an ultrasound probe for example.

The patient monitoring system in the present example further includes a display 80 operably coupled to the ultrasound processing unit of the base station 72 for displaying results of the analysis procedure performed, e.g. displaying a visual representation of the one or more second output signals.

The patient monitoring system 70 may further include a controller adapted to control acquisition of ultrasound data by a connected transducer unit in use.

The controller may control transmit and receive circuits of the ultrasound transducer unit to acquire the ultrasound signals representative of different depths. The controller may control durations of, and timings between, transmit pulses and receive windows. The controller may control gating of the input Doppler signal data over defined time windows to thereby separate different input signal channels corresponding to different depths within the subject's tissue.

In some examples said controller may be comprised locally within the ultrasound transducer unit, or the control steps performed by it may be performed locally at the ultrasound transducer unit.

As mentioned above, the ultrasound transducer unit may comprise the ultrasound processing unit. It may be an ultrasound probe unit for instance incorporating one or more ultrasound transducers and an ultrasound processing unit operatively coupled with the processing unit. The ultrasound transducer unit may locally perform at least a subset of the ultrasound data pre-processing steps and/or control steps described above.

The patient monitoring system may take different forms to that described above. For example the patient monitoring system may comprise a monitoring station (e.g. a trolley-type monitoring station), comprising a display, and being connectable with an ultrasound transducer unit.

In any example, the patient monitoring system may be connectable with any number of further sensors or data sources for monitoring the same patient or different patients.

Examples in accordance with a further aspect of the invention provide an ultrasound apparatus comprising: an ultrasound processing unit in accordance with any example or embodiment outlined above or described below, or in accordance with any claim of this application; and one or more ultrasound transducers, operatively coupled to the ultrasound processing unit, for providing the input Doppler ultrasound data to the ultrasound processing unit.

The apparatus may for example comprise an ultrasound probe unit, the probe unit incorporating the ultrasound processing unit and the one or more ultrasound transducers. For instance, the probe may comprise a housing incorporating the one or more ultrasound transducers and the ultrasound processing unit.

Examples in accordance with a further aspect of the invention provide an ultrasound processing method for use in distinguishing heart rate sources within received Doppler ultrasound data, the method comprising:

receiving input Doppler ultrasound data, including data corresponding to a plurality of different depths within a uterus region of a subject, and extracting from the data a set of input signal channels, each representative of a different tissue depth within the subject;

performing a principal component analysis, PCA, procedure, to identify one or more linear combinations of the input signal channels which are statistically uncorrelated with one another, the linear combinations defining, when composed, a set of first output signals, and

performing an independent component analysis, ICA, procedure, configured to identify one or more linear combinations of said first output signals which are statistically independent from one another, said linear combinations defining a set of second output signals.

The method may further comprise generating the set of second output signals in accordance with the identified linear combinations.

The method may further comprise processing the second output signals to derive from each a heart rate signal or heart rate measurement.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. An ultrasound processing unit, for use in fetal monitoring for distinguishing different heart rate sources within received Doppler ultrasound data, the unit configured to:

   receive (32) input Doppler ultrasound data, including data corresponding to a plurality of different depths within a uterus region of a subject, and extract (34) from the data a set of input signal channels, each representative of a different tissue depth within the subject;
   perform (36) a principal component analysis, PCA, procedure, configured to identify one or more linear combinations of the input signal channels which are statistically uncorrelated, the linear combinations defining, when composed, a set of first output signals, and
   perform (38) an independent component analysis, ICA, procedure, configured to identify one or more linear combinations of said first output signals which are statistically independent from one another, said one or more linear combinations defining a set of one or more second output signals, the one or more second output signals providing signals corresponding to distinct heart rate sources.

2. The ultrasound processing unit as claimed in claim 1, wherein the PCA procedure is configured to identify the linear combinations of said input signals that result in first output signals having a combined signal strength exceeding a defined threshold while being statistically uncorrelated with one another.

3. The ultrasound processing unit as claimed in claim 1 or 2, wherein the processing unit is further configured to generate the set of second output signals in accordance with the identified linear combinations.

4. The ultrasound processing unit as claimed in any of claim 3, wherein the processing unit is adapted to process the second output signals to derive from each a heart rate signal or heart rate measurement.

5. The ultrasound processing unit as claimed in any of claims 1-4, wherein the processing unit is further adapted to attribute to each of the second output signals a physiological source, and
optionally wherein the attribution is performed based on one or more of: an average depth within the subject to which the second output signal corresponds, a pulse rate associated with the second output signal, and a spectral content of the signal.

6. The ultrasound processing unit as claimed in any of claims 1-5, wherein the PCA procedure is configured to generate the set of first output signals in accordance with the identified linear combinations, and provide the signals as an input to the ICA procedure.

7. The ultrasound processing unit as claimed in any of claims 1-6, wherein extracting the set of input signal channels comprises gating the input Doppler ultrasound data over a plurality of different temporal windows.

8. An ultrasound apparatus comprising:

   an ultrasound processing unit as claimed in any of claims 1-7; and
   one or more ultrasound transducers, operatively coupled to the ultrasound processing unit, for providing the input Doppler ultrasound data to the ultrasound processing unit.

9. The ultrasound apparatus as claimed in claim 8, wherein the apparatus comprises an ultrasound probe unit, the probe unit incorporating the ultrasound processing unit and the one or more ultrasound transducers.

10. A patient monitoring system comprising:

    an ultrasound processing unit as claimed in any of claims 1-7; and
    a connection interface for connecting in use to an ultrasound transducer unit.

11. The patient monitoring system as claimed in claim 10, further comprising an ultrasound transducer unit coupled to said connection interface.

12. The patient monitoring system as claimed in claim 10 or 11, further including a controller adapted to control acquisition of ultrasound data by a connected transducer unit in use.

13. An ultrasound processing method for use in distinguishing different heart rate sources within received Doppler ultrasound data, the method comprising:

    receiving (32) input Doppler ultrasound data, including data corresponding to a plurality of different depths within a uterus region of a subject, and extracting (34) from the data a set of input signal channels, each representative of a different tissue depth within the subject;

performing (36) a principal component analysis, PCA, procedure, to identify one or more linear combinations of the input signal channels which are statistically uncorrelated with one another, the linear combinations defining, when composed, a set of first output signals, and

performing (38) an independent component analysis, ICA, procedure, configured to identify one or more linear combinations of said first output signals which are statistically independent from one another, said linear combinations defining a set of second output signals.

14. The method as claimed in claim 13, further comprising generating the set of second output signals in accordance with the identified linear combinations.

15. The method as claimed in claim 14, further comprising

processing the second output signals to derive from each a heart rate signal or heart rate measurement; and/or

attributing to each of the second output signals a physiological source.

FIG. 1

FIG. 2

FIG. 3

FIG. 4

FIG. 5

FIG. 6

Europäisches
Patentamt

European
Patent Office

Office européen
des brevets

# EUROPEAN SEARCH REPORT

**Application Number**

EP 19 17 0857

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2013/158407 A1 (KABAKOV SERGUEI [US] ET AL) 20 June 2013 (2013-06-20) * paragraphs [0018] - [0022], [0025] - [0032]; figures * | 1-15 | INV. A61B8/02 A61B8/08 |
| A | US 4 984 576 A (SCHULENBERG ANDREAS [DE] ET AL) 15 January 1991 (1991-01-15) * column 1, line 33 - column 2, line 20; claim 1; figure 1 * | 1-15 | |
| A | US 2014/276070 A1 (KABAKOV SERGUEI [US] ET AL) 18 September 2014 (2014-09-18) * the whole document * | 1-15 | |
| A | KRIBECHE A ET AL: "Separating fetal doppler signals in pregnancy using independent component analysis : application to the extraction of fetal heart rate and global movement", ULTRASONICS SYMPOSIUM, 2005 IEEE ROTTERDAM, THE NETHERLANDS 18-21 SEPT. 2005, PISCATAWAY, NJ, USA,IEEE, vol. 2, 18 September 2005 (2005-09-18), pages 1319-1322, XP010899087, DOI: 10.1109/ULTSYM.2005.1603096 ISBN: 978-0-7803-9382-0 * the whole document * | 1-15 | TECHNICAL FIELDS SEARCHED (IPC) A61B |
| A | US 2005/267376 A1 (MAROSSERO DOROTHEE [US] ET AL) 1 December 2005 (2005-12-01) * paragraphs [0089] - [0098] * | 1-15 | |
| A | US 2016/022164 A1 (BROCKWAY MARINA [US] ET AL) 28 January 2016 (2016-01-28) * paragraphs [0001], [0005], [0011] - [0013], [0099] * | 1-15 | |

The present search report has been drawn up for all claims

| Place of search | Date of completion of the search | Examiner |
|---|---|---|
| Munich | 29 October 2019 | Küster, Gunilla |

EPO FORM 1503 03.82 (P04C01)

**ANNEX TO THE EUROPEAN SEARCH REPORT
ON EUROPEAN PATENT APPLICATION NO.**

EP 19 17 0857

This annex lists the patent family members relating to the patent documents cited in the above-mentioned European search report.
The members are as contained in the European Patent Office EDP file on
The European Patent Office is in no way liable for these particulars which are merely given for the purpose of information.

29-10-2019

| Patent document cited in search report | | Publication date | Patent family member(s) | | Publication date |
|---|---|---|---|---|---|
| US 2013158407 | A1 | 20-06-2013 | CN 103169498 A | | 26-06-2013 |
| | | | US 2013158407 A1 | | 20-06-2013 |
| US 4984576 | A | 15-01-1991 | DE 3518967 A1 | | 27-11-1986 |
| | | | DE 3664864 D1 | | 14-09-1989 |
| | | | EP 0204192 A1 | | 10-12-1986 |
| | | | JP H0331056 B2 | | 02-05-1991 |
| | | | JP S61272035 A | | 02-12-1986 |
| | | | US 4984576 A | | 15-01-1991 |
| US 2014276070 | A1 | 18-09-2014 | CN 105208938 A | | 30-12-2015 |
| | | | EP 2967489 A1 | | 20-01-2016 |
| | | | US 2014276070 A1 | | 18-09-2014 |
| | | | WO 2014159348 A1 | | 02-10-2014 |
| US 2005267376 | A1 | 01-12-2005 | EP 1776041 A2 | | 25-04-2007 |
| | | | US 2005267376 A1 | | 01-12-2005 |
| | | | US 2005267377 A1 | | 01-12-2005 |
| | | | WO 2005117692 A2 | | 15-12-2005 |
| US 2016022164 | A1 | 28-01-2016 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4403184 A

**Non-patent literature cited in the description**

- **APPO HYVÄRINEN ; JUHA KARHUNEN ; ERKKI OJA.** Independent Component Analysis. John Wiley & Sons, Inc, 2001